# EUROPÄISCHE PATENTSCHRIFT

(11) **EP 0 944 704 B1**
(45) Veröffentlichungstag und Bekanntmachung des Hinweises auf die Patenterteilung: **12.03.2003**
(21) Anmeldenummer: 97952858.5
(22) Anmeldetag: 02.12.1997
(51) Int. Cl.: C11D 3/386

(54) **UMHÜLLTE ENZYMZUBEREITUNG MIT VERBESSERTER LÖSLICHKEIT**
COATED ENZYME PREPARATION WITH AN IMPROVED SOLUBILITY
PREPARATION D'ENZYMES ENROBES A SOLUBILITE AMELIOREE

(30) Priorität: 11.12.1996 DE 19651446
(43) Veröffentlichungstag der Anmeldung: 29.09.1999
(73) Patentinhaber: Henkel Kommanditgesellschaft auf Aktien, 40589 Düsseldorf-Holthausen (DE)
(72) Erfinder: PAATZ, Kathleen, D-40589 Düsseldorf (DE); RÄHSE, Wilfried, D-40589 Düsseldorf (DE); PICHLER, Werner, A-6250 Kundl (AT); KOTTWITZ, Beatrix, D-40593 Düsseldorf (DE)
(86) Internationale Anmeldenummer: EP9706744
(87) Internationale Veröffentlichungsnummer: WO98026037

(56) Entgegenhaltungen:
- EP-A- 0 206 418
- WO-A-91/06638
- WO-A-93/07260
- DE-A- 4 310 506
- DE-A- 4 322 229
- DATABASE WPI Section Ch, Week 8930 Derwent Publications Ltd., London, GB; Class B07, AN 89-217408 XP002066911 & JP 01 155 938 A (SHOWA DENKO KK)
- DATABASE WPI Section Ch, Week 8812 Derwent Publications Ltd., London, GB; Class D16, AN 88-080330 XP002066912 & JP 63 032 485 A (AMANO PHARM KK)

## Beschreibung

Die vorliegende Erfindung betrifft ein Enzymgranulat, ein Verfahren zu seiner Herstellung und die Verwendung des Granulats in festen Wasch- und Reinigungsmitteln.

Enzyme, insbesondere Proteasen, finden ausgedehnte Verwendung in Wasch-, Waschhilfs- und Reinigungsmitteln. Üblicherweise kommen die Enzyme dabei nicht als Konzentrate, sondern in Mischungen mit einem Verdünnungs- und Trägermaterial zum Einsatz Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DT 16 17 190 beziehungsweise durch Aufkleben mit nichtionischen Tensiden gemäß der deutschen Offenlegungsschrift DT 16 17 188 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Marumerisieren in die gewünschte Partikelform überführt, wie zum Beispiel in der deutschen Patentschrift DE 16 17 232, der deutschen Offenlegungsschrift DT 20 32 768 und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im Waschgut verfangen und dieses verunreinigen beziehungsweise sie werden ungenutzt in das Abwasser überführt. Aus der deutschen Offenlegungsschrift DT 18 03 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzten, das entstandene Gemisch zu granulieren und gegebenenfalls das Granulat mit filmbildenden Polymeren und Farbstoffen zu umhüllen. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind. Außerdem weisen die derart hergestellten Granulate eine so hohe Lösungs- beziehungsweise Zerfallsgeschwindigkeit unter Waschbedingungen auf, daß die Granulate teilweise schon bei der Lagerung relativ rasch zerfallen und die Enzyme desaktiviert werden.

Aus der europäischen Anmeldung EP 206418 sind Enzymgranulate bekannt, deren Coating als wesentliche Komponente ein alkalisches Puffer-Salz enthält, um sie gegen Bleiche-Partikel zu schützen.

Aus der internationalen Patentanmeldung WO 92/11347 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch derartige Zuschlagstoffe wird die Enzymverarbeitung ohne größere Aktivitätsverluste möglich.

Aus der internationalen Patentanmeldung WO 93/07263 ist ein Enzyme enthaltendes Granulat bekannt, das aus einem wasserlöslichen oder -dispergierbaren Kern, der mit einem Vinylpolymer überzogen ist, besteht, auf dem sich eine Schicht aus Enzym und Vinylpolymer befindet, wobei das Granulat einen Außenüberzug aus Vinylpolymer aufweist. Der Außenüberzug kann auch Pigmente enthalten. Durch den Mehrschichtenaufbau ist ein derartiges Enzymgranulat jedoch relativ aufwendig in der Herstellung.

Aus der internationalen Patentanmeldung WO 95/02031 & DE-A-4322229 sind umhüllte Enzymgranulate bekannt, deren Umhüllungsschicht aus einem Umhüllungssystem besteht, welches 30 Gew.-% bis 50 Gew.-% feinteiliges anorganisches Pigment, 45 Gew.-% bis 60 Gew.-% eines bei Raumtemperatur festen Alkohols mit einem Schmelzpunkt im Bereich von 45 °C bis 65 °C, bis zu 15 Gew.-% Emulgator für den Alkohol, bis zu 5 Gew.-% Dispergiermittel für das Pigment und bis zu 3 Gew.-% Wasser enthält. Durch die Anwesenheit relativ hoher Mengen wasserunlöslichen Fettalkohols können derartige Enzymgranulate zu Rückstandsproblemen bei der Auflösung in Wasser führen, da die Anwesenheit des Emulgators oft nicht ausreicht, die organischen Anteile des Umhüllungssystems in Lösung zu bringen.

In der internationalen Patentanmeldung WO 93/07260 wird ein Herstellungsverfahren für staubfreie Enzymgranulate offenbart, welches das Aufsprühen einer Fermentationsbrühe auf ein hydratisierbares Trägermaterial, anschließendes Aufsprühen einer Lösung bestimmter Umhüllungsmaterialien, zu denen Fettsäureester, alkoxylierte Alkohole, Polyvinylalkohol, Polyethylenglykol, Zucker und Stärke gehören, und Verdampfen des Lösungsmittels umfaßt.

Auch in der internationalen Patentanmeldung WO 91/06638 wird ein Verfahren zum Coating von Enzymgranulaten offenbart, nach welchem zur Bildung der äußersten Schicht ein wasserlösliches oder -dispergierbares Agens, beispielsweise PEG 8000 zusammen mit TiO₂ als wäßrige Lösung beziehungsweise Suspension aufgesprüht wird.

Die in den genannten Dokumenten eingesetzten Überzugsmassen für die äußerste Umhüllungsschicht werden normalerweise in Form einer wäßrigen Dispersion in einem Wirbelschichttrockner auf das Enzymgranulat aufgebracht. Dabei besteht zum einen die Gefahr der zumindest oberflächlichen Zerstörung der Granulate durch Staubabrieb in der Wirbelschicht, was zu einem erhöhtem Anteil an äußerst feinkörnigem Material im Enzymgranulat führt, welches für die Zumischung zu üblichen partikelförmigen Wasch - oder Reinigungsmitteln unbrauchbar ist, da es sich nicht homogen in der entstehenden Mischung verteilt und überdies enzymhaltiger Feinstaub zu allergenen Reaktionen beim Waschmittel-Anwender führen kann. Daher ist man bestrebt, den Feinkornanteil im Enzymgranulat so gering wie möglich zu halten, um möglichst wenig durch Aussieben oder Windsichten entfernen zu müssen. Zum anderen ist der Einsatz des Umhüllungsmaterials in gelöster Form nachteilig, da in einem separaten Schritt das auf das Enzymgranulat aufgebrachte Lösungsmittel wieder entfernt werden muß.

Es bestand daher die Aufgabe, ein Umhüllungssystem zu entwickeln, das bei gleichmäßigem Auftrag auf ein enzymhaltiges Granulat der oberflächlichen Zerstörung des Granulats entgegenwirkt, die Lagerstabilität des Enzyms durch schützende Umhüllung des gesamten Granulats erhöht, in der Waschflotte ein rasches und vollständiges Lösungsverhalten des Granulats gewährleistet, die Überdeckung eventueller Eigenfärbung des nicht umhüllten Enzymgranulats erlaubt und eventuell störenden Geruch des nicht umhüllten Granulats, wahrscheinlich durch Verhinderung der Diffusion der Geruchsstoffe an die Enzymgranulatoberfläche, beseitigt; insbesondere die Löslichkeit der beschichteten Granulate sollte verbessert werden.

Dies wurde erfindungsgemäß durch ein für die Einarbeitung in insbesondere teilchenförmige Wasch- oder Reinigungsmittel geeignetes Enzymgranulat, enthaltend Enzym und organisches und/oder anorganisches Trägermaterial und eine gleichmäßige äußere pigmenthaltige Umhüllungsschicht gelöst, welches dadurch gekennzeichnet ist, daß die äußere Umhüllungsschicht aus einem Umhüllungssystem besteht, das (a) 5 Gew.-% bis 70 Gew.-% feinteiliges anorganisches wasserunlösliches Pigment, (b) 45 Gew.-% bis 90 Gew.-% bei Raumtemperatur festen wasserlöslichen organischen Stoff mit einem Schmelzpunkt im Bereich von 40 °C bis 70 °C und (c) bis zu 20 Gew.-% Rieselfähigkeitsverbesserer enthält, wobei der bei Raumtemperatur feste wasserlösliche Stoff (b) ein mit durchschnittlich 45 bis 120, insbesondere 60 bis 110 Molequivalenten Ethylenoxid veretherter primärer linearer gesättigter oder ungesättigter Alkohol mit 16 bis 22 C-Atomen, ein ethoxyliertes Fettsäureamid, ein Ethoxylierungsprodukt von Fettsäureestern oder Hydroxyfettsäureestern mit 1 bis 6 C-Atomen im Alkoholteil des Esters mit einem jeweiligen Ethoxylierungsgrad von vorzugsweise 45 bis 120 oder ein Gemisch aus diesen ist.

Die Erfindung betrifft weiterhin ein Verfahren zur Herstellung eines für die Einarbeitung in partikelförmige Wasch- oder Reinigungsmittel geeigneten Enzymgranulates mit einer mittleren Korngröße von 0,8 mm bis 1,4 mm durch Extrudieren eines durch Vermischen einer wäßrigen Enzymflüssigkeit, die eine gegebenenfalls durch Mikrofiltration von unlöslichen Bestandteilen befreite, aufkonzentrierte Fermentationsbrühe sein kann, mit anorganischem und/oder organischem Trägermaterial als Zuschlagstoff entstandenen Enzym-Vorgemischs, Sphäronisierung des Extrudats in einem Rondiergerät, gegebenenfalls Trocknung und Aufbringen einer äußeren Umhüllungsschicht, wobei man in einer Wirbelschicht aus Extrudat eine äußere Umhüllungsschicht eines Überzugssystems aufbringt, das (a) 5 Gew.-% bis 70 Gew.-%, insbesondere 10 Gew.-% bis 50 Gew.-% feinteiliges anorganisches wasserunlösliches Pigment, (b) 45 Gew.-% bis 90 Gew.-%, insbesondere 50 Gew.-% bis 85 Gew.-% bei Raumtemperatur festen wasserlöslichen organischen Stoff mit einem Schmelzpunkt im Bereich von 40 °C bis 70 °C und (c) bis zu 20 Gew.-%, insbesondere 1 Gew.-% bis 5 Gew.-% Rieselfähigkeitsverbesserer enthält, wobei der bei Raumtemperatur feste wasserlösliche Stoff (b) ein mit durchschnittlich 45 bis 120, insbesondere 60 bis 110 Molequivalenten Ethylenoxid veretherter primärer linearer gesättigter oder ungesättigter Alkohol mit 16 bis 22 C-Atomen, ein ethoxyliertes Fettsäureamid, ein Ethoxylierungsprodukt von Fettsäureestern oder Hydroxyfettsäureestem mit 1 bis 6 C-Atomen im Alkoholteil des Esters mit einem jeweiligen Ethoxylierungsgrad von vorzugsweise 45 bis 120 oder ein Gemisch aus diesen ist.

Unter wasserlöslichen Stoffen werden im Rahmen der vorliegenden Schrift Substanzen verstanden, die sich bei einer Temperatur von 30 °C zu mindestens 50 g/l, insbesondere mindestens 80 g/l in Wasser lösen. Fettalkohole gehören demnach nicht zu den wasserlöslichen Stoffen.

Die Hauptkomponente des Überzugssystems, der wasserlösliche bei Raumtemperatur feste Stoff, wird insbesondere aus alkoxylierten Alkoholen, Fettsäureestern, Fettsäureamiden und/oder Hydroxyfettsäureestern ausgewählt. Es handelt sich vorzugsweise um einen Alkohol, insbesondere einen primären linearen Alkohol, mit 16 bis 22 C-Atomen. der mit 45 bis 120, insbesondere 60 bis 110 Molequivalenten Alkylenoxid, insbesondere Ethylenoxid, verethert ist. Zu den genannten Alkoholen gehören insbesondere Stearylalkohol, Arachidylalkohol, Behenylalkohol und ein- bis dreifach ungesättigte Alkohole entsprechender Kettenlänge, wobei wesentlich ist, daß die genannte alkoxylierte Alkoholkomponente einen Schmelzpunkt im Bereich von 40 °C bis 70 °C, insbesondere von 50 °C bis 60 °C aufweist, worunter hier die Temperatur verstanden werden soll, bei der beim Erwärmen 100 % des Stoffs in flüssiger Form vorliegen. Alternativ oder zusätzlich zu den Alkoholethoxylaten können auch ethoxylierte Fettsäureamide und/oder Ethoxylierungsprodukte von Fettsäureestern oder Hydroxyfettsäureestern mit 1 bis 6 C-Atomen im Alkoholteil des Esters, beispielsweise Ricinolsäureglycerid, wobei der Ethoxylierungsgrad jeweils vorzugsweise 45 bis 120, insbesondere 60 bis 110 beträgt, eingesetzt werden. Die Fettsäurekomponente der genannten Substanzen besitzt vorzugsweise 12 bis 22, insbesondere 16 bis 18 C-Atome. Zu den in diesem Zusammenhang bevorzugten Alkoxylaten gehören Ethoxylierungsprodukte mit sogenannter eingeengter Homologenverteilung (nre, "narrow range ethoxylates"), wie sie gemäß dem Verfahren der europäischen Patentschrift EP 339 426 oder der internationalen Patentanmeldung WO 90/13533 erhalten werden. Gewünschtenfalls können die Ethoxygruppen in den genannten Alkoxylierungsprodukten zumindest teilweise durch Propoxygruppen ersetzt sein. Bei Einsatz von Stoffgemischen sind auch solche brauchbar, welche geringe Anteile, normalerweise unter 15 Gew.-% bezogen auf das Gemisch, an bei Raumtemperatur flüssigen Anteilen enthalten, solange das gesamte Gemisch bei Raumtemperatur fest erscheint und einen Erstarrungspunkt im Bereich von 40 °C bis 70 °C, insbesondere von 50 °C bis 60 °C aufweist. Der Erstarrungspunkt ist die Temperatur, bei der beim Abkühlen auf eine Temperatur oberhalb des Schmelzpunkts erwämten Materials eine Verfestigung eintritt. Er kann mit Hilfe eines rotierenden Thermometers nach dem Verfahren der DIN ISO 2207 bestimmt werden. Für das erfindungsgemäße Herstellverfahren besonders geeignet sind Substanzen, welche in Abmischung mit den übrigen Komponenten des Umhüllungssystems eine möglichst homogene, bei Temperaturen bis zu 120 °C versprühbare Schmelze ergeben. Als Anhaltspunkt kann in diesem Zusammenhang dienen, daß Flüssigkeiten mit Viskositäten bis zu etwa 500 mPa·s bei den genannten Temperaturen in der Regel problemlos mittels dafür vorgesehener Vorrichtungen, wie zum Beispiel aus der deutschen Patentanmeldung DE 196 44 244 bekannt, versprüht und auf Enzymgranulate aufgebracht werden können.

Vorzugsweise sind als zusätzliche Komponente des Umhüllungsmaterials untergeordnete Mengen, bevorzugt 3 Gew.-% bis 45 Gew.-%, insbesondere 4 Gew.-% bis 35 Gew.-% einer Verbindung gemäß allgemeiner Formel I,

R[O(CₙH₂ₙO)ₘH]ₓ (I)

in der R einen organischen Rest mit mindestens 2 C-Atomen, vorzugsweise 3 bis 12 C-Atomen und insbesondere 4 bis 10 C-Atomen, n 2 oder 3, m 1 bis 15 und x 2 oder 3 bedeutet, enthalten. Derartige Komponenten können in bekannter Weise durch Umsetzung von Alkoholen R[OH]ₓ mit Ethylenoxid und/oder Propylenoxid hergestellt werden und können Teil des oben erwähnten bei Raumtemperatur flüssigen Anteils sein. Zu den bevorzugten Verbindungen gemäß allgemeiner Formel I gehören solche, in denen sowohl Ethoxygruppen (n=2) als auch 1,2-Propoxygruppen (n=3) enthalten sind, wobei die durchschnittliche Anzahl von Ethoxygruppen pro Hydroxylgruppe des Alkohols R[OH]ₓ vorzugsweise bis zu 10 und die durchschnittliche Anzahl von Propoxygruppen pro Hydroxylgruppe des Alkohols R[OH]ₓ vorzugsweise bis zu 5 beträgt. Unter diesen werden solche bevorzugt eingesetzt, bei deren Herstellung der genannte Alkohol zuerst mit Propylenoxid und anschließend mit Ethylenoxid umgesetzt worden ist Zu den bevorzugten Alkoholen R[OH]ₓ gehören 1,6-Hexylenglykol, Glycerin und Trimethylolpropan, obwohl auch 1,2-Hydroxyethan brauchbar ist.

In einer bevorzugten Ausführungsform der Erfindung handelt es sich bei dem Umhüllungsmaterialsystem um eine Mischung aus 10 Gew.-% bis 35 Gew.-% wasserunlöslichem feinteiligem anorganischem Pigment, 20 Gew.-% bis 77 Gew.-%, insbesondere 30 Gew.-% bis 71 Gew.-% obengenanntem ethoxyliertem Fettalkohol und 3 Gew.-% bis 45 Gew.-%, insbesondere 4 Gew.-% bis 35 Gew.-% Verbindung nach allgemeiner Formel I.

Zu den wasserunlöslichen anorganischen Pigmenten, mit denen eventuelle störende Färbungen des Enzymgranulats überdeckt werden können, gehören beispielsweise Calciumcarbonat, Titandioxid, welches in Rutil- oder Anatase-Kristallmodifikation vorliegen kann, Zinkoxid, Zinksulfid, Bleiweiß (basisches Bleicarbonat), Bariumsulfat, Aluminiumhydroxid, Antimonoxid, Lithopone (Zinksulfid-Bariumsulfat), Kaolin, Kreide, Talkum und/oder Glimmer. Diese liegen in so feinteiliger Form vor, daß sie in einer Schmelze der übrigen Bestandteile des Überzugssystems dispergiert werden können. Üblicherweise liegt die mittlere Teilchengröße derartiger Pigmente im Bereich von 0,004 µm bis 50 µm. Auch der Einsatz von mit Dispergiermitteln oberflächenmodifizierten Pigmenten ist möglich. Vorzugsweise wird mit Al-, Si-, Zr- oder Polyol-Verbindungen oberflächenmodifiziertes Titandioxidpigment, insbesondere in Rutilform, wie es beispielsweise unter den Handelsnamen Kronos® 2132 (Fa. Kronos-Titan) oder Hombitan® R 522 (Sachtleben Chemie GmbH) vertrieben wird, eingesetzt. Brauchbar sind auch die Tiona® RLL-, AG- beziehungsweise VC-Typen der Fa. Solvay sowie die Bayertitan® RD-, R-KB- und AZ-Typen der Fa. Bayer AG.

Als weitere Komponente des Überzugssystems kommen Rieselfahigkeitsverbesserer in Frage. Darunter sollen Wirkstoffe verstanden werden, deren Abwesenheit zu einer Verschlechterung der Rieselfähigkeit des umhüllten Granulats führt. Brauchbar sind zum Beispiel Aluminiumsilikate, Zeolithe, Natriumsilikate oder Kieselsäuren, die in feinteiliger Form zum Aufbringen auf das Enzmgranulat homogen mit den übrigen Bestandteilen des Umhüllungssystems vermischt werden oder separat nach Aufbringen der übrigen Bestandteile des Umhüllungssystems aufgebracht werden können.

Als Enzyme kommen in erster Linie die aus Mikroorganismen, wie Bakterien oder Pilzen, gewonnenen Proteasen, Lipasen, Amylasen und/oder Cellulasen in Frage, wobei von Bacillus-Arten erzeugte Proteasen, allein oder in Kombination mit Amylasen, bevorzugt sind. Sie werden in bekannter Weise durch Fermentationsprozesse aus geeigneten Mikroorganismen gewonnen, die zum Beispiel in den deutschen Offenlegungsschriften DE 19 40 488, DE 20 44 161, DE 21 01 803 und DE 21 21 397, den US-amerikanischen Patentschriften US 3 632 957 und US 4 264 738 sowie der europäischen Patentanmeldung EP 006 638 beschrieben sind. Besonders vorteilhaft kann das erfindungsgemäße Verfahren zur Konfektierung von sehr aktiven Proteasen, die beispielsweise aus der internationalen Patentanmeldung WO 91/2792 bekannt sind, angewendet werden, weil deren lagerstabile Einarbeitung in Wasch- und Reinigungsmittel oft Probleme bereitet und erfindungsgemäß die Entstehung unerwünschter Enzymstäube vermieden wird. Enzyme sind in den erfindungsgemäßen Granulaten vorzugsweise in Mengen von 4 Gew.-% bis 20 Gew.-% enthalten. Falls es sich bei dem erfindungsgemäßen Enzymgranulat um eine proteasehaltige Formulierung handelt, beträgt die Proteaseaktivität vorzugsweise 150 000 Proteaseeinheiten (PE, bestimmt nach der in Tenside 7 (1970), 125 beschriebenen Methode) bis 350 000 PE, insbesondere 160 000 PE bis 300 000 PE, pro Gramm Enzymgranulat.

Als Trägermaterialien für das Enzym sind im Prinzip alle organischen oder anorganischen pulverförmigen Substanzen brauchbar, welche die zu granulierenden Enzyme nicht oder nur tolerierbar wenig zerstören oder desaktivieren und unter Granulationsbedingungen stabil sind. Zu derartigen Substanzen gehören beispielsweise Stärke, Getreidemehl, Cellulosepulver, Alkalialumosilikat, insbesondere Zeolith, Schichtsilikat, zum Beispiel Bentonit oder Smectit, und wasserlösliche anorganische oder organische Salze, zum Beispiel Alkalichlorid, Alkalisulfat, Alkalicarbonat oder Alkaliacetat, wobei Natrium oder Kalium die bevorzugten Alkalimetalle sind. Bevorzugt wird ein Trägermaterialgemisch eingesetzt, das in Wasser quellfähige Stärke sowie gegebenenfalls Getreidemehl, Cellulosepulver und/oder Alkalicarbonat enthält.

Bei der in Wasser quellfähigen Stärke handelt es sich vorzugsweise um Maisstärke, Reisstärke, Kartoffelstärke oder Gemische aus diesen, wobei der Einsatz von Maisstärke besonders bevorzugt ist. Quellfähige Stärke ist in den erfindungsgemäßen Enzymgranulaten vorzugsweise in Mengen von 20 Gew.-% bis 50 Gew.-%, insbesondere von 25 Gew.-% bis 45 Gew.-% enthalten.

Bei dem gegebenenfalls enthaltenen Getreidemehl handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird dabei ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten quellfähigen Stärken führenden Getreidearten ist möglich, wenn darauf geachtet wird, daß die Mehle aus den ganzen Körnern hergestellt worden sind. Durch die Mehlkomponente des Zuschlagstoffgemisches wird bekanntermaßen eine wesentliche Geruchsreduzierung der Enzymzubereitung erreicht, welche die Geruchsverminderung durch die Einarbeitung gleicher Mengen entsprechender Stärkearten bei weitem übertrifft. Derartiges Getreidemehl ist in den erfindungsgemäßen Enzymgranulaten vorzugsweise in Mengen bis zu 35 Gew.-%, insbesondere von 15 Gew.-% bis 25 Gew.-% enthalten.

Die erfindungsgemäßen Enzymgranulate enthalten als weitere Komponente des Trägermaterials vorzugsweise 1 Gew.-% bis 50 Gew.-%, insbesondere 5 Gew.-% bis 25 Gew.-%, bezogen auf gesamtes Granulat, eines Granulierhilfsmittelsystems, das Alkali-Carboxymethylcellulose mit Substitutionsgraden von 0,5 bis 1 und Polyethylenglykol und/oder Alkylpolyethoxylat enthält. In diesem Granulierhilfsmittelsystem sind vorzugsweise, jeweils bezogen auf fertiges Enzymgranulat, 0,5 Gew.-% bis 5 Gew.-% Alkali-Carboxymethylcellulose mit Substitutionsgraden von 0,5 bis I und bis zu 3 Gew.-% Polyethylenglykol und/oder Alkylpolyethoxylat enthalten, wobei besonders bevorzugt ist, wenn mindestens 0,5 Gew.-%, insbesondere 0,8 Gew.-% bis 2 Gew.-% Polyethylenglykol mit einer mittleren Molmasse unter 1000 und/oder Alkylpolyethoxylat mit mindestens 30 Ethoxygruppen vorhanden ist, falls mehr als 2 Gew.-% Alkali-Carboxymethylcellulose enthalten ist. Höher substituierte Carboxymethylcellulose, mit Substitutionsgraden bis zu 3, ist in dem Granulierhilfsmittelsystem vorzugsweise nicht enthalten.

Auch phosphatierte, gegebenenfalls teilhydrolysierte Stärken kommen als Granulierhilfsmittel in Frage. Unter phosphatierter Stärke wird ein Stärkederivat verstanden, bei der Hydroxylgruppen der Stärke-Anhydroglukoseeinheiten durch die Gruppe -O-P(O)(OH)₂ oder deren wasserlösliche Salze, insbesondere Alkalisalze wie Natrium- und/oder Kaliumsalze, ersetzt sind. Unter dem mittleren Phosphatierungsgrad der Stärke ist die Zahl der veresterten, eine Phosphatgruppe tragenden Sauerstoffatome pro Saccharid-Monomer der Stärke gemittelt über alle Saccharid-Einheiten zu verstehen. Der mittlere Phosphatierungsgrad bei vorzugsweise eingesetzten phosphatierten Stärken liegt im Bereich von 1,5 bis 2,5, da besonders bei deren Einsatz viel geringere Mengen erforderlich sind, um eine bestimmte Granulatfestigkeit zu erreichen, als bei Einsatz von Carboxymethylcellulose. Unter teilhydrolysierten Stärken sollen im Rahmen der vorliegenden Erfindung Oligo- beziehungsweise Polymere von Kohlenhydraten verstanden werden, die nach üblichen, beispielsweise säure- oder enzymkatalysierten Verfahren durch partielle Hydrolyse von Stärke zugänglich sind. Vorzugsweise handelt es sich um Hydrolyseprodukte mit mittleren Molmassen im Bereich von 440 bis 500 000. Bevorzugt sind Poiysaccharide mit einem Dextrose-Equivalent (DE) im Bereich von 0,5 bis 40, insbesondere von 2 bis 30, wobei DE ein gebräuchliches Maß für die reduzierende Wirkung eines Polysaccharids im Vergleich zu Dextrose, welche ein DE von 100 besitzt, ist. Brauchbar sind nach Phosphatierung sowohl Maltodextrine (DE 3 - 20) und Trockenglukosesirupe (DE 20-37) als auch sogenannte Gelbdextrine und Weißdextrine mit höheren mittleren Molmassen im Bereich von etwa 2000 bis 30 000. Bezogen auf fertiges Granulat sind Gehalte von 0,1 Gew.-% bis 20 Gew.-%, insbesondere 0,5 Gew.-% bis 15 Gew.-% phosphatierter Stärke bevorzugt.

Gegebenenfalls können als zusätzliche Bestandteile des Granulierhilfsmittelsystems auch weitere Cellulose- oder Stärkeether, wie Carboxymethylstärke, Methylcellulose, Hydroxyethylcellulose, Hydroxypropylcellulose sowie entsprechende Cellulosemischether, Gelatine, Casein, Traganth, Maltodextrose, Saccharose, Invertzucker, Glukosesirup oder andere in Wasser lösliche beziehungsweise gut dispergierbare Oligomere oder Polymere natürlichen oder synthetischen Ursprungs verwendet werden. Brauchbare synthetische wasserlösliche Polymere sind Polyacrylate, Polymethacrylate, Copolymere der Acrylsäure mit Maleinsäure oder vinylgruppenhaltige Verbindungen, ferner Polyvinylalkohol, teilverseiftes Polyvinylacetat und Polyvinylpyrrolidon. Soweit es sich bei den vorgenannten Verbindungen um solche mit freien Carboxylgruppen handelt, liegen sie normalerweise in Form ihrer Alkalisalze, insbesondere ihrer Natriumsalze vor. Derartige zusätzliche Granulierhilfsmittel können in den erfindungsgemäßen Enzymgranulaten in Mengen bis zu 10 Gew.-%, insbesondere von 0,5 Gew.-% bis 8 Gew.-% enthalten sein. Höhermolekulare Polyethylenglykole, das heißt solche mit einem mittleren Molekulargewicht über 1000, sind zwar als synthetische wasserlösliche Polymere mit staubbindender Wirkung brauchbar, doch bewirken gerade die höhermolekularen Polyethylenglykole eine unerwünschte Erhöhung der benötigten Granulatauflösezeit, so daß diese Substanzen in den erfindungsgemäßen Enzymgranulaten vorzugsweise völlig fehlen.

Zur Herstellung der erfindungsgemäßen Enzymgranulate geht man vorzugsweise von Fermentbrühen aus, die beispielsweise durch Mikrofiltration von unlöslichen Begleitsfoffen befreit werden können. Die Mikrofiltration wird dabei vorzugsweise als Querstrom-Mikrofiltration unter Verwendung poröser Rohre mit Mikroporen größer 0,1 mm, Fließgeschwindigkeiten der Konzentratlösung von mehr als 2 m/s und einem Druckunterschied zur Permeatseite von unter 5 bar durchgeführt, wie beispielsweise in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration kann dabei, wie in der internationalen Patentanmeldung WO 92/11347 beschrieben, so geführt werden, daß man nur zu relativ niedrigen Gehalten an Trockensubstanz von vorzugsweise 5 Gew.-% bis 50 Gew.-%, insbesondere von 10 Gew.-% bis 40 Gew.-% gelangt. Das Konzentrat wird einem zweckmäßigerweise zuvor hergestellten trockenen, pulverförmigen bis körnigen Gemisch der oben beschriebenen Zuschlagstoffe zudosiert. Der Wassergehalt der Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Das rieselfähige Vorgemisch wird im Prinzip bekannter Weise anschließend in einem Kneter sowie einem angeschlossenen Extruder zu einer plastischen, möglichst homogenen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,7 mm bis 1,2 mm, vorzugsweise 0,8 mm bis 1,0 mm. Die in dieser Form vorliegenden Partikel können anschließend getrocknet und mit dem Überzugssystem gemäß der Erfindung umhüllt werden. Es hat sich jedoch als vorteilhaft erwiesen, die den Extruder und Zerhacker verlassenden zylindrischen Partikel vor dem Umhüllen zu sphäronisieren, das heißt sie in geeigneten Vorrichtungen abzurunden und zu entgraten. Man verwendet hierzu eine Vorrichtung, die aus einem zylindrischen Behälter mit stationären, festen Seitenwänden und einer bodenseitig drehbar gelagerten Reibplatte bestehen. Vorrichtungen dieser Art sind unter der Warenbezeichnung Marumerizer® in der Technik verbreitet und beispielsweise in den deutschen Auslegeschriften DE 21 37 042 und DE 21 37 043 beschrieben. Anschließend können eventuell auftretende staubformige Anteile mit einer Korngröße unter 0,1 mm, insbesondere unter 0,4 mm sowie eventuelle Grobanteile mit einer Korngröße über 2 mm, insbesondere über 1,6 mm durch Sieben oder Windsichten entfernt und gegebenenfalls in den Herstellungsprozess zurückgeführt werden. Nach der Sphäronisierung werden die Kügelchen kontinuierlich oder chargenweise, vorzugsweise unter Verwendung einer Wirbelschichttrockenanlage, bei Zulufttemperaturen von vorzugsweise 35 °C bis 50 °C und insbesondere bei einer Produkttemperatur von nicht über 42 °C bis zum gewünschten Restfeuchtegehalt von beispielsweise 4 Gew.-% bis 10 Gew.-%, insbesondere 5 Gew.-% bis 8 Gew.-%, bezogen auf gesamtes Granulat, getrocknet, falls sie zuvor höhere Wassergehalte aufwiesen.

Anstatt, nach oder vorzugsweise während der Trocknung wird das Überzugssystem gemäß der Erfindung als äußere Umhüllung aufgebracht. In einer bevorzugten Ausgestaltung des erfindungsgemäßen Herstellungsverfahrens wird das Überzugssystem, gegebenenfalls unter Kühlung, als erwärmte, bei einer Temperatur von 5 °C bis 45 °C über dem Schmelzpunkt der bei Raumtemperatur festen organischen wasserlöslichen Komponente vorliegende Flüssigkeit auf das Extrudat aufgebracht. Vorzugsweise bringt man, bezogen auf fertiges Granulat, 5 Gew.-% bis 25 Gew.-% des Überzugssystems als äußere Umhüllungsschicht auf das enzymhaltige Extrudat auf.

Die nach dem erfindungsgemäßen Verfahren erhaltene Enzymzubereitung besteht aus weitgehend abgerundeten, gleichmäßig umhüllten und staubfreien Partikeln, die in der Regel ein Schüttgewicht von etwa 500 bis 900 Gramm pro Liter, insbesondere 650 bis 880 Gramm pro Liter aufweisen. Die erfindungsgemäßen Granulate zeichnen sich durch eine sehr hohe Lagerstabilität, insbesondere bei Temperaturen über Raumtemperatur und hoher Luftfeuchtigkeit, sowie ein rasches und vollständiges Lösungsverhalten in der Waschflotte aus. Vorzugsweise setzen die erfindungsgemäßen Granulate 100% ihrer Enzymaktivität innerhalb von 3 Minuten, insbesondere innerhalb von 90 Sekunden bis 2 Minuten, in Wasser bei 25 °C frei.

Das erfindungsgemäße oder nach dem erfindungsgemäßen Verfahren hergestellte Enzymgranulat wird vorzugsweise zur Herstellung fester, insbesondere teilchenförmiger Waschoder Reinigungsmittel verwendet, die durch einfaches Vermischen der Enzymgranulate mit in derartigen Mitteln üblichen weiteren Pulverkomponenten erhalten werden können. Für die Einarbeitung in teilchenförmige Wasch- und Reinigungsmittel weist das Enzymgranulat vorzugsweise mittlere Korngrößen im Bereich von 0,8 mm bis 1,2 mm auf. Die erfindungsgemäßen Granulate enthalten vorzugsweise weniger als 2 Gew.-%, insbesondere höchstens 1,4 Gew.-% an Partikeln mit Korngrößen außerhalb des Bereichs von 0,4 mm bis 1,6 mm.

### Beispiele

### Beispiel 1

Ein nach der Fermentation gewonnener Erntebrei, wie in der internationalen Patentanmeldung WO 91/2792 beschrieben, mit 75 000 Proteaseeinheiten pro g (PE/g) wurde nach der Entfernung der Fermentationsrückstände durch Dekantieren und Mikrofiltration in einer Ultrafiltrationsanlage aufkonzentriert. Nach der weiteren Aufkonzentrierung mittels Vakuumeindampfung enthielt die wäßrige Enzymsuspension 700000 PE/g. Dieses Proteasekonzentrat wurde mit Zuschlagstoffen (6 Gew.-% Saccharose, 4 Gew.-% Cellulose, 5 Gew.-% Carboxymethylcellulose mit Substitutionsgrad 0,65-0,75, 16 Gew.-% Weizenmehl, 36 Gew.-% Maisstärke und 3 Gew.-% Polyethylenglykol mit mittlerem Molgewicht 2000, Mengenangaben jeweils bezogen auf entstehendes Gemisch) vermischt, homogenisiert und anschließend in einem Extruder mit Schneidevorrichtung in Granulate überführt. Der Lochdurchmesser der Lochplatte des Extruders betrug 0,85 mm. Das Verhältnis von Länge zu Dicke des Granulatkorns lag bei 1. Nach der Verrundung und Trocknung der Granulate wurden die Partikel mit Teilchengröße kleiner 0,4 mm und größer 1,6 mm abgesiebt. Die Kornfraktion zwischen 0,4 mm und 1,6 mm wurde in einem Rotorgranulator des Typs GPCG-5 der Fa. Glatt mit einer Coatingschicht überzogen, wobei eine Coatingschmelze aus 70 Gew.-% 80 fach ethoxyliertem C_{16/18}-Fettalkohol (Lutensol® AT 80; Hersteller BASF) und 30 Gew.-% Titandioxid zum Einsatz kam. 16 Gew.-%, bezogen auf entstehendes Enzymgranulat, der bei 120 °C gehaltenen Schmelze wurden auf das Enzymgranulat unter folgenden Betriebsbedingungen aufgesprüht:

| | |
|---|---|
| Einsatzmenge an Enzymgranulat | 10 kg |
| Zulufttemperatur | 40 °C |
| Produkttemperatur | 42 °C |
| Ablufttemperatur | 41 °C |
| Luftmenge | 150 m³/h |
| Rotor | 260 Umdrehungen/Minute |
| Zweistoffdüse-Sprühlufttemperatur | 120°C |
| Dosierrate für Coatingschmelze | 50 g/Minute. |

Das Coatingmaterial bildete auf der Granulatoberfläche einen gleichmäßigen, unporösen Film aus. Zur Bestimmung des Staubabriebes wurden 60 g des so hergestellten Granulats **P1** in eine Wirbelschicht gegeben. Die Abluft der Wirbelschicht strömte durch einen Filter. Die nach 40 Minuten Verweilzeit des Enzymgranulats unter diesen Bedingungen aufgefangene Staubmenge entspricht der Staubabriebmenge. Im vorliegenden Fall war der Staubabrieb mit unter 10 mg pro Filter vernachlässigbar gering.

### Beispiel 2

Beispiel 1 wurde wiederholt, wobei nun eine Coatingsschmelze bestehend aus 20 Gew.-% Titandioxid, 48 Gew.-% 80 fach ethoxyliertem C_{16/18}-Fettalkohol, 25 Gew.-% Polyethylenglykol und 7 Gew.-% Trimethylolpropan, das mit 3 Equivalenten Propylenoxid und 7 Equivalenten Ethylenoxid pro Hydroxylgruppe umgesetzt worden war, zum Einsatz kam. Auch hierbei erhielt man ein Granulat (**P2**) mit vernachlässigbar geringem Staubabrieb

### Beispiel 3

Zur Bestimmung des rückstandsfreien Auflösens der Enzymgranulate wurden 1000 ml Wasser (16 °dH, bei 30 °C temperiert) in ein 2000 ml-Becherglas (hohe Form) gegeben, ein Laborrührer mit Propellerrührkopf wurde 1,5 cm vom Becherglasboden entfernt zentriert fixiert und mit 800 Umdrehungen pro Minute in Bewegung gesetzt. 8 g des zu testenden Granulats wurden eingestreut und 90 Sekunden gerührt. Anschließende wurde die im Becherglas befindliche Flüssigkeit durch ein Sieb (Maschenweite 0,2 mm) mit bekanntem Gewicht gegossen, das Becherglas mit möglichst wenig kaltem Wasser nachgespült und das Sieb nach Trocknen bei 40 °C bis zur Gewichtskonstanz ausgewogen. Es ergaben sich die in Tabelle 2 angegebenen Siebrückstände (Doppelbestimmung), wobei neben den erfindungsgemäßen Granulaten **P1** und **P2** zum Vergleich ein Enzymgranulat **V1** des Standes der Technik, welches mit der gleichen Menge Coatingmaterial, die aber aus 78 Gew.-% C₁₈-Fettalkohol, 4 % 40-fach ethoxyliertem C_{16/18}-Fettalkohol und 18 Gew.-% Titandioxid bestand, getestet wurde.

**Tabelle 2:**

| Siebrückstände der Enzymgranulate | |
|---|---|
| Enzymgranulat | Siebrückstand [Gew.-%] |
| **P1** | 2,5 |
| **P2** | 2,5 |
| **V1** | 96,0 |

## Patentansprüche

1. Für die Einarbeitung in insbesondere teilchenförmige Wasch- oder Reinigungsmittel geeignetes Enzymgranulat, enthaltend Enzym und anorganisches und/oder organisches Trägermaterial und eine gleichmäßige äußere pigmenthaltige Umhüllungsschicht, welches **dadurch gekennzeichnet ist, daß** die äußere Umhüllungsschicht aus einem Umhüllungssystem besteht, das (a) 5 Gew.-% bis 70 Gew.-% feinteiliges anorganisches wasserunlösliches Pigment, (b) 45 Gew.-% bis 90 Gew.-% bei Raumtemperatur festen wasserlöslichen organischen Stoff mit einem Schmelzpunkt im Bereich von 40 °C bis 70 °C und (c) bis zu 20 Gew.-% Rieselfähigkeitsverbesserer enthält, wobei der bei Raumtemperatur feste wasserlösliche Stoff (b) ein mit durchschnittlich 45 bis 120, insbesondere 60 bis 110 Molequivalenten Ethylenoxid veretherter primärer linearer gesättigter oder ungesättigter Alkohol mit 16 bis 22 C-Atomen, ein ethoxyliertes Fettsäureamid, ein Ethoxylierungsprodukt von Fettsäureestern oder Hydroxyfettsäureestem mit 1 bis 6 C-Atomen im Alkoholteil des Esters mit einem jeweiligen Ethoxylierungsgrad von vorzugsweise 45 bis 120 oder ein Gemisch aus diesen ist.

2. Enzymgranulat nach Anspruch 1, **dadurch gekennzeichnet, daß** das Umhüllungssystem 50 Gew.-% bis 85 Gew.-% des bei Raumtemperatur festen wasserlöslichen organischen Stoffes (b) enthält.

3. Enzymgranulat nach Anspruch 1 oder 2, **dadurch gekennzeichnet, daß** das Umhüllungssystem 10 Gew.-% bis 50 Gew.-% feinteiliges anorganisches wasserunlösliches Pigment (a) enthält.

4. Enzymgranulat nach einem der Ansprüche 1 bis 3, **dadurch gekennzeichnet, daß** das Umhüllungssystem bis zu 10 Gew.-%, insbesondere 1 Gew.-% bis 5 Gew.-% Rieselfähigkeitsverbesserer (c) enthält

5. Enzymgranulat nach einem der Ansprüche 1 bis 4, **dadurch gekennzeichnet, daß** als zusätzliche Komponente (d) des Umhüllungsmaterials 3 Gew.-% bis 45 Gew.-% einer Verbindung gemäß allgemeiner Formel I,
R[O(CₙH₂ₙO)ₘH]ₓ (I)
in der R einen organischen Rest mit mindestens 2 C-Atomen, bevorzugt 3 bis 12 C-Atomen und insbesondere 4 bis 10 C-Atomen, n 2 oder 3, m 1 bis 15 und x 2 oder 3 bedeutet, enthalten sind.

6. Enzymgranulat nach Anspruch 5, **dadurch gekennzeichnet, daß** die Verbindungen gemäß Formel I durch Umsetzung von Alkoholen R[OH]ₓ mit Ethylenoxid und/oder Propylenoxid hergestellt worden und in ihnen sowohl Ethoxygruppen (n=2) als auch 1,2-Propoxygruppen (n=3) enthalten sind, wobei die durchschnittliche Anzahl von Ethoxygruppen pro Hydroxylgruppe des Alkohols R[OH]ₓ insbesondere bis zu 10 und die durchschnittliche Anzahl von Propoxygruppen pro Hydroxylgruppe des Alkohols R[OH]ₓ insbesondere bis zu 5 beträgt.

7. Enzymgranulat nach einem der Ansprüche 1 bis 6, **dadurch gekennzeichnet, daß** das Umhüllungssystem eine Mischung aus 10 Gew.-% bis 35 Gew.-% feinteiligem anorganischem wasserunlöslichem Pigment (a), 20 Gew.-% bis 77 Gew.-%, insbesondere 30 Gew.-% bis 71 Gew.-% ethoxyliertem Fettalkohol (b) und 3 Gew.-% bis 45 Gew.-%, insbesondere 4 Gew.-% bis 35 Gew.-% Komponente (d) nach allgemeiner Formel I ist.

8. Enzymgranulat nach einem der Ansprüche 1 bis 7, **dadurch gekennzeichnet, daß** die Umhüllungsschicht als feinteiliges anorganisches wasserunlösliches Pigment (a) Calciumcarbonat, Titandioxid, Zinkoxid, Zinksulfid, Bleiweiß, Bariumcarbonat, Bariumsulfat, Aluminiumhydroxid, Antimonoxid, Kaolin, Kreide, Talkum und/oder Glimmer enthält.

9. Enzymgranulat nach einem der Ansprüche 1 bis 8, **dadurch gekennzeichnet, daß** es Protease, Amylase, Lipase und/oder Cellulase enthält

10. Enzymgranulat nach einem der Ansprüche 1 bis 9, **dadurch gekennzeichnet, daß** es Protease mit einer Aktivität von 150 000 PE bis 350 000 PE, insbesondere 160 000 PE bis 300 000 PE, pro Gramm Enzymgranulat enthält.

11. Verfahren zur Herstellung eines für die Einarbeitung in partikelförmige Wasch- oder Reinigungsmittel geeigneten Enzymgranulates mit einer mittleren Korngröße von 0,8mm bis 1,4mm durch Extrudieren eines durch Vermischen einer wäßrigen Enzymflüssigkeit mit anorganischem und/oder organischem Trägermaterial als Zuschlagstoff entstandenen Enzym-Vorgemischs, Sphäronisierung des Extrudats in einem Rondiergerät und Aufbringen einer äußeren Umhüllungsschicht, wobei man in einer Wirbelschicht aus Extrudat eine äußere Umhüllungsschicht eines Überzugssystems aufbringt, das (a) 5 Gew.-% bis 70 Gew.-%, insbesondere 10 Gew.-% bis 50 Gew.-% feinteiliges anorganisches wasserunlösliches Pigment, (b) 45 Gew.-% bis 90 Gew.-%, insbesondere 50 Gew.-% bis 85 Gew.-% bei Raumtemperatur festen wasserlöslichen organischen Stoff mit einem Schmelzpunkt im Bereich von 40 °C bis 70 °C und (c) bis zu 20 Gew.-%, insbesondere 1 Gew.-% bis 5 Gew.-% Rieselfähigkeitsverbesserer enthält, wobei der bei Raumtemperatur feste wasserlösliche Stoff (b) ein mit durchschnittlich 45 bis 120, insbesondere 60 bis 110 Molequivalenten Ethylenoxid veretherter primärer linearer gesättigter oder ungesättigter Alkohol mit 16 bis 22 C-Atomen, ein ethoxyliertes Fettsäureamid, ein Ethoxylierungsprodukt von Fettsäureestern oder Hydroxyfettsäureestern mit 1 bis 6 C-Atomen im Alkoholteil des Esters mit einem jeweiligen Ethoxylierungsgrad von vorzugsweise 45 bis 120 oder ein Gemisch aus diesen ist.

12. Verfahren nach Anspruch 11, **dadurch gekennzeichnet, daß** die wäßrige Enzymflüssigkeit eine gegebenenfalls durch Mikrofiltration von unlöslichen Bestandteilen befreite, aufkonzentrierte Fermentationsbrühe ist.

13. Verfahren nach Anspruch 11 oder 12, **dadurch gekennzeichnet, daß** man, bezogen auf fertiges Granulat, 5 Gew.-% bis 25 Gew.-% des Überzugssystems als äußere Umhüllungsschicht auf das enzymhaltige Extrudat aufbringt.

14. Verfahren nach einem der Ansprüche 11 bis 13, **dadurch gekennzeichnet, daß** man das Überzugssystem als Flüssigkeit, die bei einer Temperatur von 5 °C bis 45 °C über dem Schmelzpunkt des bei Raumtemperatur festen wasserlöslichen organischen Stoffes (b) vorliegt, auf das Extrudat aufbringt.

15. Verwendung eines Enzymgranulats gemäß einem der Ansprüche 1 bis 10 oder erhältlich nach dem Verfahren gemäß einem der Ansprüche 11 bis 14 zur Herstellung fester, insbesondere teilchenförmiger Wasch- oder Reinigungsmittel.

## Claims

1. Enzyme granules suitable for incorporation in detergents, especially particulate detergents, containing enzyme and inorganic and/or organic carrier material and a uniform outer pigment-containing coating layer, **characterized in that** the outer coating layer consists of a coating system containing (a) 5% by weight to 70% by weight of fine-particle inorganic water-insoluble pigment, (b) 45% by weight to 90% by weight of a water-soluble organic substance solid at room temperature with a melting point in the range from 40°C to 70°C and (c) up to 20% by weight of flow improvers, the water-soluble substance (b) solid at room temperature being a primary linear saturated or unsaturated C₁₆₋₂₂ alcohol etherified with on average 45 to 120 and, more particularly, 60 to 110 mol equivalents of ethylene oxide, an ethoxylated fatty acid amide, an ethoxylation product of fatty acid esters or hydroxyfatty acid esters containing 1 to 6 carbon atoms in the alcohol part of the ester and having a degree of ethoxylation of preferably 45 to 120 or a mixture thereof.

2. Enzyme granules as claimed in claim 1, **characterized in that** the coating system contains 50% by weight to 85% by weight of the water-soluble organic substance (b) solid at room temperature.

3. Enzyme granules as claimed in claim 1 or 2, **characterized in that** the coating system contains 10% by weight to 50% by weight of fine-particle inorganic water-insoluble pigment (a).

4. Enzyme granules as claimed in any of claims 1 to 3, **characterized in that** the coating system contains up to 10% by weight and, more particularly, 1% by weight to 5% by weight of flow improvers (c).

5. Enzyme granules as claimed in any of claims 1 to 4, **characterized in that** 3% by weight to 45% by weight of a compound corresponding to general formula I:
R[O(CₙH₂ₙO)ₘH]ₓ (I)
in which R is an organic radical containing at least 2 carbon atoms, preferably 3 to 12 carbon atoms and more preferably 4 to 10 carbon atoms, n is the number 2 or 3, m is a number of 1 to 15 and x has a value of 2 or 3, are present as an additional component (d) of the coating material.

6. Enzyme granules as claimed in claim 5, **characterized in that** the compounds corresponding to formula I are prepared by reaction of alcohols R[OH]ₓ with ethylene oxide and/or propylene oxide and contain both ethoxy groups (n = 2) and 1,2-propoxy groups (n = 3), the average number of ethoxy groups per hydroxyl group of the alcohol R[OH]ₓ being in particular up to 10 and the average number of propoxy groups per hydroxyl group of the alcohol R[OH]ₓ being in particular up to 5.

7. Enzyme granules as claimed in any of claims 1 to 6, **characterized in that** the coating system is a mixture of 10% by weight to 35% by weight of fine-particle water-insoluble inorganic pigment (a), 20% by weight to 77% by weight and, more particularly, 30% by weight to 71% by weight of ethoxylated fatty alcohol (b) and 3% by weight to 45% by weight and, more particularly, 4% by weight to 35% by weight of a compound (d) corresponding to general formula I.

8. Enzyme granules as claimed in any of claims 1 to 7, **characterized in that** the coating layer contains calcium carbonate, titanium dioxide, zinc oxide, zinc sulfide, white lead, barium carbonate, barium sulfate, aluminium hydroxide, antimony oxide, kaolin, chalk, talcum and/or mica as the fine-particle inorganic water-insoluble pigment (a).

9. Enzyme granules as claimed in any of claims 1 to 8, **characterized in that** they contain protease, amylase, lipase and/or cellulase.

10. Enzyme granules as claimed in any of claims 1 to 9, **characterized in that** they contain protease with an activity of 150,000 PU to 350,000 PU and, more particularly, 160,000 PU to 300,000 PU per gram of enzyme granules.

11. A process for the production of enzyme granules suitable for incorporation in particulate detergents with a mean particle size of 0.8 mm to 1.4 mm by extruding an enzyme premix formed by mixing an aqueous enzyme liquid with an inorganic and/or organic carrier material as additive, spheronizing the extrudate in a spheronizer and applying an outer coating layer, an outer coating layer of a coating system containing (a) 5% by weight to 70% by weight and more particularly 10% by weight to 50% by weight of fine-particle inorganic water-insoluble pigment, (b) 45% by weight to 90% by weight and more particularly 50% by weight to 85% by weight of a water-soluble organic substance solid at room temperature with a melting point in the range from 40°C to 70°C and (c) up to 20% by weight and more particularly 1% by weight to 5% by weight of flow improvers, the water-soluble substance (b) solid at room temperature being a primary linear saturated or unsaturated C₁₆₋₂₂ alcohol etherified with on average 45 to 120 and, more particularly, 60 to 110 mol equivalents of ethylene oxide, an ethoxylated fatty acid amide, an ethoxylation product of fatty acid esters or hydroxyfatty acid esters containing 1 to 6 carbon atoms in the alcohol part of the ester and having a degree of ethoxylation of preferably 45 to 120 or a mixture thereof,
being applied in a fluidized bed of extrudate.

12. A process as claimed in claim 11, **characterized in that** the aqueous enzyme liquid is a concentrated fermentation broth optionally freed from insoluble constituents by microfiltration.

13. A process as claimed in claim 11 or 12, **characterized in that**, based on the final granules, 5% by weight to 25% by weight of the coating system is applied to the enzyme-containing extrudate as an outer coating layer.

14. A process as claimed in any of claims 11 to 13, **characterized in that** the coating system is applied to the extrudate as a liquid which is present at a temperature of 5°C to 45°C above the melting point of the water-soluble organic substance (b) solid at room temperature.

15. The use of the enzyme granules claimed in any of claims 1 to 10 or obtainable by the process claimed in any of claims 11 to 14 for the production of solid, more particularly particulate, detergents.

## Revendications

1. Granulé d'enzyme approprié pour l'incorporation dans des produits de lavage ou de nettoyage en particulier sous forme particulaire, contenant un enzyme et un matériau de support anorganique et/ou organique et une couche d'enrobage contenant un pigment, externe, homogène
**caractérisé en ce que**
la couche d'enrobage externe consiste en système d'enrobage qui contient :
a) de 5 % en poids à 70 % en poids de pigment anorganique insoluble dans l'eau, finement divisé,
b) de 45 % en poids à 90 % en poids, de substance organique soluble dans l'eau, solide à température ambiante ayant un point de fusion dans la zone de 40°C à 70°C ,et
c) jusqu'à 20 % en poids d'un agent améliorant l'aptitude à l'écoulement,
dans lequel la substance (b), soluble dans l'eau solide à la température ambiante est un alcool saturé ou non saturé, linéaire, primaire, éthérifié par en moyenne 45 à 120 en particulier de 60 à 110 équivalents molaires d'oxyde d'éthylène, ayant de 16 à 22 atomes de carbone, un amide d'acide gras éthoxylé, un produit d'éthoxylation d'esters, d'acides gras ou d'esters d'acide gras hydroxylé ayant de 1 à 6 atomes de carbone dans la partie alcool de l'ester avec un degré d'éthoxylation respectif de préférence de 45 à 120, ou un mélange de ceux-ci.

2. Granulé d'enzyme selon la revendication 1,
**caractérisé en ce que**
le système d'enrobage renferme de 50 % en poids à 85 % en poids de la substance (b) organique soluble dans l'eau, solide à température ambiante.

3. Granulé d'enzyme selon la revendication 1 ou 2,
**caractérisé en ce que**
le système d'enrobage renferme de 10% en poids à 50% en poids de pigment (a) insoluble dans l'eau anorganique finement divisé.

4. Granulé d'enzyme selon l'une des revendications 1 à 3,
**caractérisé en ce que**
le système d'enrobage renferme jusqu'à 10 % en poids, - en particulier de 1 % en poids à 5 % en poids d'agent améliorant la capacité d'écoulement (c).

5. Granulé d'enzyme selon l'une des revendications 1 à 4,
**caractérisé en ce que**
comme composant supplémentaire (d) de matériau d'enrobage il contient de 3 % en poids à 45 % en poids d'un composé conformément à la formule générale I
R[(O(Cₙ H₂ₙO)ₘ H]ₓ (I)
dans laquelle R signifie un reste organique ayant au moins 2 atomes de carbone, de préférence de 3 à 12 atomes de carbone, et en particulier de 4 à 10 atomes de carbone, n signifie 2 ou 3, m signifie 1 à 15, et x signifie 2 ou 3.

6. Granulé d'enzyme selon la revendication 5,
**caractérisé en ce que**
les composés de formule I ont été préparés par réaction d'alcools R[OH]ₓ avec l'oxyde d'éthylène et/ou l'oxyde de propylène, et contiennent aussi bien des groupes éthoxy (n=2) que des groupes 1,2-propoxy (n=3), dans lesquels le nombre moyen de groupes éthoxy par groupe hydroxyle de l'alcool R[OH]ₓ s'élève en particulier à jusqu'à 10 et le nombre moyen des groupes propoxy par groupe hydroxyle de l'alcool R[OH]ₓ s'élève en particulier jusqu'à 5.

7. Granulé d'enzyme selon l'une des revendications 1 à 6, **caractérisé en ce que**
le système d'enrobage est un mélange à base de 10 % en poids à 35 % en poids de pigment (a) anorganique, insoluble dans l'eau, finement divisé, de 20 % en poids à 77 % en poids, - en particulier de 30 % en poids à 71 % en poids d'alcool gras éthoxylé (b) et de 3 % en poids a 45 % en poids, - en particulier de 4 % en poids à 35 % en poids de composant (d) selon la formule générale I.

8. Granulé d'enzyme selon l'une des revendications 1 à 7, **caractérisé en ce que**
la couche d'enrobage contient comme pigment (a) anorganique insoluble dans l'eau, finement divisé, du carbonate de calcium, du dioxyde de titane, de l'oxyde de zinc, du sulfure de zinc, du blanc de plomb, du carbonate de baryum, du sulfate de baryum, de l'hydroxyde d'aluminium, de l'oxyde d'antimoine, du Kaolin, de la craie, du talc et/ou du mica.

9. Granulé d'enzyme selon l'une des revendications 1 à 8,
**caractérisé en ce qu'**
il contient de la protéase, de l'amylase, de la lipase et/ou de la cellulase.

10. Granulé d'enzyme selon l'une des revendication 1 à 9,
**caractérisé en ce que**
il contient des protéases ayant une activité de 150 000 PE à 350 000 PE, - en particulier de 160 000 PE à 300 000 PE par gramme de granulé d'enzyme.

11. Procédé de préparation d'un granulé d'enzyme approprié pour l'incorporation dans des produits de lavage ou de nettoyage sous forme particulaire ayant une taille de grains en moyenne de 0,8 mm à 1,4 mm par extrusion d'un prémélange d'enzyme formé par mélange d'un liquide aqueux d'enzyme avec un matériau de support anorganique et/ou organique comme additif, sphéronisation de l'extrudat dans un appareil pour arrondir et application d'une couche d'enrobage externe, dans lequel on applique dans une couche tourbillonnaire en extrudat, une couche d'enrobage externe d'un système de revêtement qui contient.
(a) de 5 % en poids à 70 % en poids en particulier de 10 % en poids à 50 % en poids de pigment insoluble dans l'eau anorganique, finement divisé,
(b) de 45 % en poids à 90 % en poids en particulier de 50 % en poids à 85 % en poids d'une substance organique soluble dans l'eau, solide à température ambiante ayant un point de fusion dans la zone de 40°C à 70 °C, et
c) jusqu'à 20 % en poids - en particulier de 1 % en poids à 5 % en poids d'agent améliorant la capacité d'écoulement,
pour lequel la substance (b) soluble dans l'eau, solide à température ambiante, est un alcool saturé ou non saturé, linéaire, primaire éthérifié avec en moyenne 45 à 120, - en particulier de 60 à 110 équivalents molaires d'oxyde d'éthylène, ayant de 16 à 22 atomes de carbone un amide d'acide gras ethoxylé, un produit d'éthoxylation d'esters d'acide gras ou d'ester d'acide gras hydroxylé, ayant de 1 à 6 atomes de carbone dans la fraction alcool de l'ester avec un degré d'éthoxylation respectif de préférence de 45 à 120 ou un mélange à base de ceux ci.

12. Procédé selon la revendication 11,
**caractérisé en ce que**
le liquide aqueux d'enzyme est un bouillon de fermentation concentré, débarrassé éventuellement par microfiltration, des constituants insolubles .

13. Procédé selon la revendication 11 ou la revendication 12,
**caractérisé en ce qu'**
on applique de 5 % en poids à 25 % en poids rapporté au granulé terminé de système de revêtement comme couche externe d'enrobage sur l'extrudat contenant un enzyme.

14. Procédé selon l'une des revendications 11 à 13,
**caractérisé en ce qu'**
on applique le système de revêtement sous forme de liquide qui se présente à une température de 5 °C à 45 °C au dessus du point de fusion de la substance (b) organique soluble dans l'eau, solide à température ordinaire, sur l'extrudat.

15. Utilisation d'un granulé d'enzyme conformément à l'une des revendications 1 à 10 ou accessible selon le procédé conformément à l'une des revendications 11 à 14, en vue de la production de produits de lavage ou de nettoyage solides, en particulier sous forme particulaire.
